# EUROPEAN PATENT APPLICATION

(11) **EP 2 789 307 A1**
(43) Date of publication of application: **15.10.2014**
(21) Application number: 14163760.3
(22) Date of filing: 07.04.2014
(51) Int. Cl.: A61B 18/14, A61B 17/32, A61B 17/3211, A61B 17/00

(54) **Surgical device for pediatric surgery**

(30) Priority: 08.04.2013 US 201361809454 P; 04.04.2014 US 201414245022
(71) Applicant: Covidien LP, Mansfield, MA 02048 (US)
(72) Inventor: Marczyk, Stanislaw, Stratford, CT Connecticut 06614 (US); Switalski, Christopher, Glastonbury, CT 06033 (US); Pribanic, Russell, Roxbury, CT Connecticut 06783 (US)
(74) Representative: Soames, Candida Jane

(57) **Abstract**

A surgical device is provided for use in pediatric surgery and includes a dissection blade moveably mounted in a sheath of the surgical device. The surgical device includes an electrical connection for supply cauterizing current to the blade. A handle assembly is provided and includes an advancement mechanism for incrementally advancing the blade out of the sheath and a lever to release the blade from the advancement mechanism. A lock member is provided to maintain the blade in an extended condition.

## Description

### CROSS-REFERENCE TO RELATED APPLICATIONS

This application claims the benefit of and priority to U.S. Provisional Patent Application No. 61/809,454, filed April 8, 2013, the entire disclosure of which is incorporated by reference herein.

### BACKGROUND

### 1. Technical field

The present disclosure relates to a surgical dissection and grasping device. More particularly, the present disclosure relates to a surgical device having a dissection blade movably mounted within a distal end of the surgical device to shield the blade prior to use.

### 2. Background Of Related Art

Pyloric stenos is a condition that affects the gastrointestinal tract of infants. It is a thickening and narrowing of the pylorus muscle in the lower part of the stomach where food or other substances pass into the small intestine. It causes vomiting and other complications such as dehydration, salt and fluid imbalances, etc. Pyloric stenos is often treated by a surgical procedure called pyloromyotomy which involves severing and spreading the thickened muscle to relax it. This procedure can be performed through open surgery or laparoscopically through a small incision or port. Since the patient is an infant the operative area is very small.

The instruments used in a pyloromyotomy are usually designed with adults in mind and typically included sheathed arthroscopic knives. Other knives in makeshift holders have also been used. These surgical instruments are often too large for the precise cuts needed with infants and, additionally, both graspers and bladed instruments were required increasing the number of instruments involved and the time to perform the surgery.

Therefore, there exists a need for a combined dissecting and tissue spreading instrument to limit the number of instruments inserted within the operative cavity of an infant. There further exists a need for a sheathed dissection instrument extendable in discrete, small increments for use on infants. There still further exists a need for a combined surgical instrument having safety mechanisms to prevent extending a dissection blade during use.

### SUMMARY

There is disclosed a surgical device for use in pediatric surgery including a handle assembly having a handle housing, an elongate tubular member extending distally from the handle assembly and having a distal end, and an end effector assembly mounted on the distal end of the elongate tubular member. The end effector assembly includes a tissue dissecting sheath and a blade movably mounted within the sheath.

The handle assembly includes an advancement mechanism for extending the blade out of the sheath. The advancement mechanism is connected to the blade by an intermediate tube rigidly affixed to the blade and movably mounted within the elongate tubular member. The advancement mechanism further includes a ratchet body connected to a proximal end of the intermediate tube and an advance button movably mounted in the handle housing. The advance button is engageable with the ratchet body to drive the ratchet body distally within the handle assembly. The advancement mechanism additionally includes an advance pawl rotatably mounted to the advance button. The ratchet body includes ratchet teeth engageable by the advance pawl to incrementally advance the ratchet body distally within the handle housing in response to depression of the advance button against the handle housing.

The advance button is pivotally mounted on the handle housing at a pivot point and the advancement mechanism further includes a lock pawl engageable with the ratchet teeth and sharing the pivot point with the advance button such that the lock pawl remains engaged with the ratchet teeth upon release of the advance button.

The surgical device further includes a cam release bar movably mounted within the handle housing and connected to a lever movably mounted on the handle housing. The cam release bar has a first cam edge engageable with the advance pawl and a second cam edge engageable with the lock pawl. Actuation of the lever drives the cam release bar distally within the housing to lift the advance pawl and lock pawl out of engagement with the ratchet teeth.

The surgical device may further include a cauterization assembly including an electrical connection assembly mounted on a proximal end of the handle housing. The electrical connection assembly supplies cauterizing current to the blade. The cauterization assembly includes a wire extending from the electrical connection assembly through the housing to the blade.

There is also disclosed a surgical device for use in pediatric surgery including a handle assembly having a handle housing and an elongate tubular member extending distally from the handle assembly and having a distal end. An end effector assembly is mounted on the distal end of the elongate tubular member and includes a sheath and a blade movably mounted within the sheath. An advancement mechanism is positioned within the handle housing and is operable to extend the blade out of the sheath. A lever is movably mounted on the handle housing and is operable to release the blade from an extended position relative to the sheath to a retracted position within the sheath. The blade is biased proximally within the sheath by a compression spring. The blade is rigidly connected to an intermediate tube and a proximal end of the intermediate tube is connected to a ratchet body movably mounted in the handle housing. The compression spring biases the ratchet body proximally within the handle housing.

The advancement mechanism includes an advance pawl engageable with the ratchet body and a lock pawl engageable with the ratchet body. A cam release bar is movably mounted in the handle housing and is engageable with the advance and lock pawls to disengage the advance and lock pawls from the ratchet body. The cam release bar is connected to the lever by a linkage.

There is further disclosed a surgical device for use in pediatric surgery including a handle assembly having a handle housing and an elongate tubular member extending distally from the handle assembly and having a distal end. An end effector assembly is mounted on the distal end of the elongate tubular member and includes a sheath and a blade movably mounted within the sheath. An advancement mechanism is positioned within the handle housing and is operable to extend the blade out of the sheath. The advancement mechanism includes a ratchet body rigidly connected to the blade. An advance button is movably mounted to the handle housing and an advance pawl is movably connected to the advance button. The advance pawl is engageable with teeth on the ratchet body. A lock pawl is also movably connected to the advance button and engageable with the ratchet body to maintain the ratchet body and blade in a distal position.

A lever is movably mounted on the handle housing and a cam release bar is connected to the lever. The lever is operable to drive the cam release bar against the lock pawl to release the blade for proximal movement within the sheath.

An electrical connector is mounted on a proximal end of the handle housing for supplying cauterizing current to the blade. A wire extends from the electrical connector to a proximal end of the intermediate tube.

### DESCRIPTION OF THE DRAWINGS

Various embodiments of the presently disclosed pediatric surgical device are disclosed herein with reference to the drawings, wherein:
FIG. 1 is a perspective view of one embodiment of a pediatric surgical device for use in pediatric surgery;
FIG. 2 is a side plan view of the handle assembly of the pediatric surgical device;
FIG. 3 is a top plan view of the handle assembly of the pediatric surgical device;
FIG. 4 is an enlarged area of detail view of FIG. 3 illustrating an indicator of the pediatric surgical device;
FIG. 5 is a perspective view of the handle assembly of the pediatric surgical device;
FIG. 6 is an enlarged area of detail view of FIG. 5 illustrating a cautery attachment point;
FIG. 7 is a perspective view of a cautery connector for use with the pediatric surgical device;
FIG. 8 is an enlarged perspective view of a proximal end of the handle assembly with the cautery connector removed and a plug assembly installed in its place;
FIG. 9 is a perspective view of the plug assembly;
FIG. 10 is an enlarged perspective view of a distal end of the pediatric surgical device of FIG. 1;
FIG. 11 is another perspective view of the distal end of the pediatric surgical device with a blade in an extended position;
FIG. 12 is a perspective view, with parts separated, of the pediatric surgical device of FIG. 1;
FIG. 13 is a perspective view, with parts separated, of the distal end of the pediatric surgical device;
FIG. 14 is a perspective view of an indicator member of the pediatric surgical device;
FIG. 15 is another perspective view of the indicator member;
FIG. 16 is a perspective view, with half of a handle housing removed, of the handle assembly of the pediatric surgical device;
FIG. 17 is an enlarged area of detail view of FIG. 16;
FIG. 18 is a cross-sectional view of the pediatric surgical device taken along line 18-18 of FIG. 1;
FIG. 19 is an enlarged area of detail view of FIG. 18;
FIG. 20 is a cross-sectional view taken along line 20-20 of FIG. 19;
FIG. 21 is an another enlarged area of detail view, shown in section, of FIG. 18;
FIG. 22 is a cross-sectional view taken along line 22-22 of FIG. 21;
FIG. 23 is a side view, shown in section, of a portion of the handle assembly including a blade advancement mechanism;
FIG. 24 is an enlarged top view of an indicator of the pediatric surgical device;
FIG. 25 is a side view, shown in section, of the distal end of the pediatric surgical device during extension of a blade;
FIG. 26 is a perspective view of the distal end during extension of the blade;
FIG. 27 is a side view, similar to FIG. 23, during release of the blade advancement mechanism;
FIG. 28 is a perspective view of the handle assembly, with half the handle housing removed, illustration actuation of a blade retraction mechanism;
FIG. 29 is an enlarged area of detail view of FIG. 28;
FIG. 30 is a side view, shown in section, of the handle assembly with the blade retraction mechanism fully actuated;
FIG. 31 is an enlarged area of detail view of FIG. 30;
FIG. 32 is a perspective view of the distal end of the pediatric surgical device, with the blade in an extended condition, being used to create an incision in a tissue;
FIG. 33 is a perspective view similar to FIG. 32 with the blade in a retracted condition;
FIG. 34 is a perspective view of the distal end of the pediatric surgical device being used to spread the incision in the tissue open;
FIG. 35 is a perspective view of the distal end of the pediatric surgical device incorporating another embodiment of a blade; and
FIG. 36 is an end view of the blade of FIG. 35.

### DETAILED DESCRIPTION OF EMBODIMENTS

Embodiments of the presently disclosed surgical device for pediatric surgery or pediatric surgical device will now be described in detail with reference to the drawings wherein like numerals designate identical or corresponding elements in each of the several views. As is common in the art, the term 'proximal" refers to that part or component closer to the user or operator, i.e. surgeon or physician, while the term "distal" refers to that part or component further away from the user.

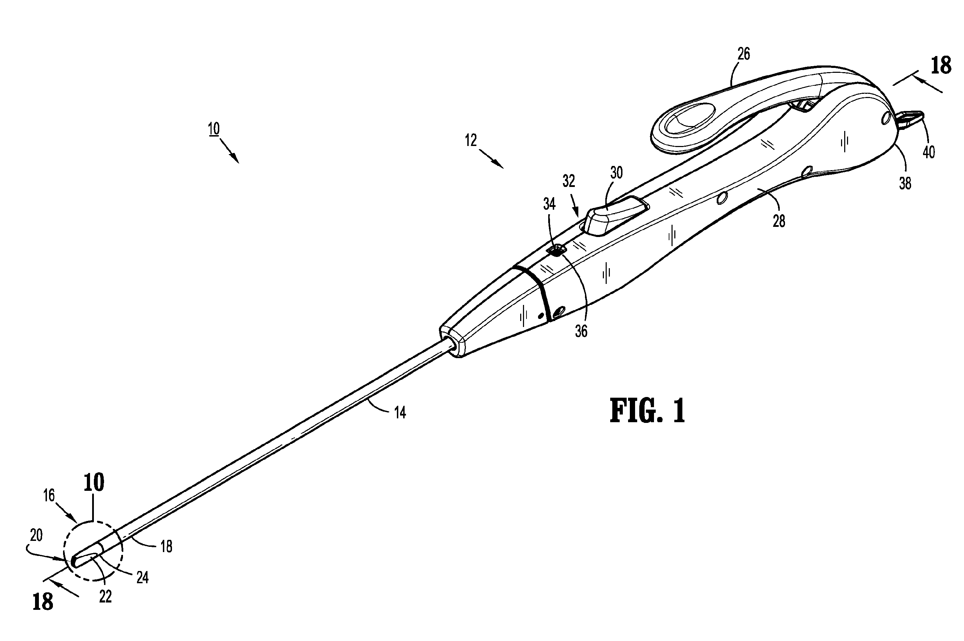

Referring initially to FIG. 1, there is disclosed one embodiment of a pediatric surgical device 10 for use in performing a pyloromyotomy procedure during open or laparoscopic surgery. Surgical device 10 generally includes a handle assembly 12, an elongate tubular member 14 extending distally from handle assembly 12 and an end effector assembly 16 mounted on a distal end 18 of elongate tubular member 14. In a specific embodiment, elongate tubular member 14 has an outer diameter of 3mm making it particularly suitable for use in pediatric surgery.

End effector assembly 16 combines multiple functions such as, for example, cutting, and dissecting and includes a blade 20 longitudinally movably mounted in a sheath 22. A proximal end 24 of sheath 22 is affixed to distal end 18 of elongate tubular member 14. Handle assembly 12 includes a lever 26 movably mounted to a handle housing 28 of handle assembly 12. Lever 26 is operable to retract blade 20 into sheath 24 in a manner described in more detail hereinbelow.

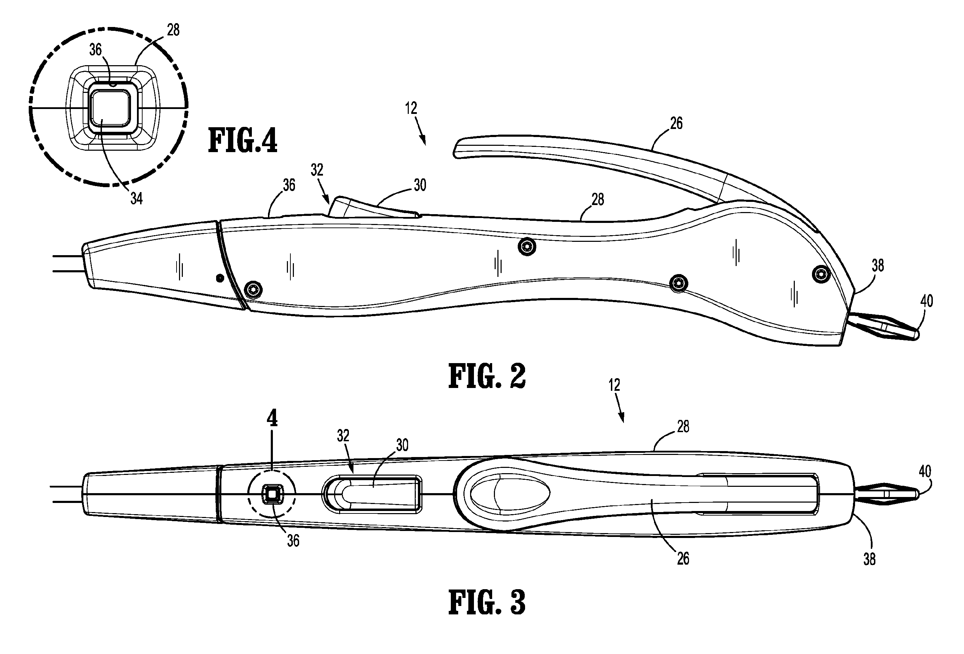

Referring now to FIGS. 1-4, in order to extend blade 20 out of sheath 22, handle assembly 12 includes an advance button 30 which is part of an advancement mechanism 32 and functions to incrementally advance blade 20 out of sheath 22 in a manner described in more detail hereinbelow. An indicator 34 is provided to give a visual indication of the extent of the extension of blade 20 out of sheath 22 and is visible through a window 36 provided in handle housing 28. Lever 26 is operable to disengage advancement mechanism 32 and allow blade 20 to retract fully within sheath 22.

In order to provide cauterization capabilities to surgical device 10, and specifically blade 20, or (when tubing 14 is insulated) to the sheath tip 22, a proximal end 38 of handle housing 28 is provided with a cauterization connection point or connector 40. Connector 40 is formed as a four arm, spring steel component. Connector 40 receives electrical input from outside sources located within the operating room such that blade 20 or tip 22 can cauterize tissue after the tissue is cut.

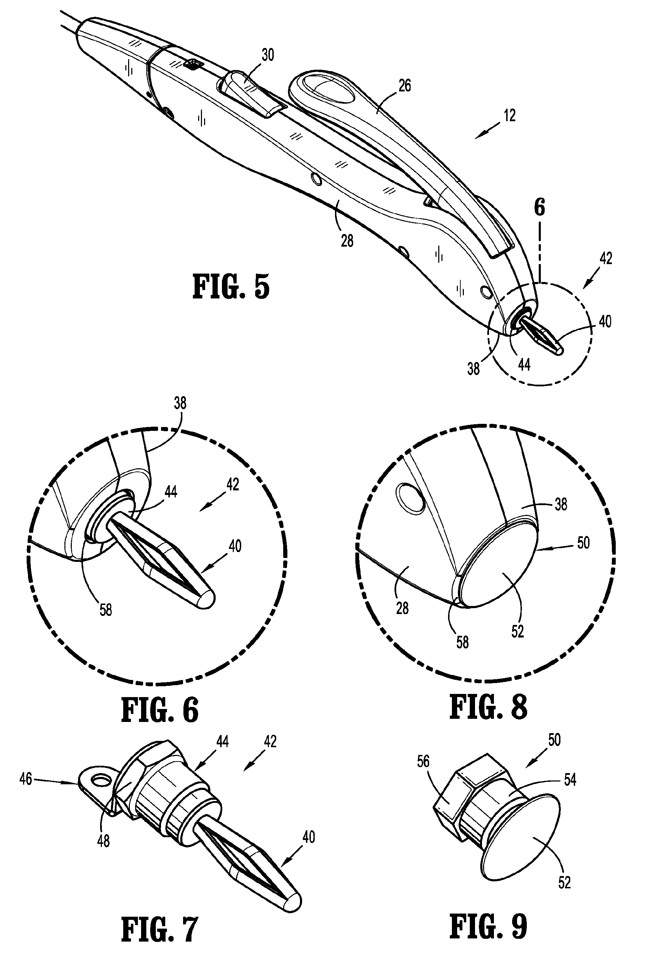

Referring specifically to FIGS. 5-9, and initially with regard to FIGS. 5-7, connector 40 is part of a connection assembly 42 having a base 44. Base 44 insulates connector 40 from handle housing 28. With specific reference to FIG. 7, connector 40 is electrically connected through insulating base 44 to a connection plate 46. Connection plate 46 supplies power to blade 20 in a manner described in more detail hereinbelow. Connection assembly 42 additionally includes a nut 48, threaded onto base 44, to secure connection assembly 42 to handle housing 28.

Referring to FIGS. 8 and 9, surgical device 10 can be supplied without cautery capability. In the absence of cauterization capabilities, a plug 50 is provided through proximal end 38 of handle housing 28. Plug 50 includes an enlarged proximal end 52 which fits flush with proximal end 38 of handle housing 28. Referring to FIG. 9, plug 50 includes a stem 54 extending distally from enlarged proximal end 52 and a nut 56 threaded onto stem 54 for securing plug 50 to handle housing 28. Plug 50 covers a hole 58 in proximal end 38 of handle housing 28 through which plug 50 and connection assembly 42 are mounted (FIGS. 6, 8 and 12).

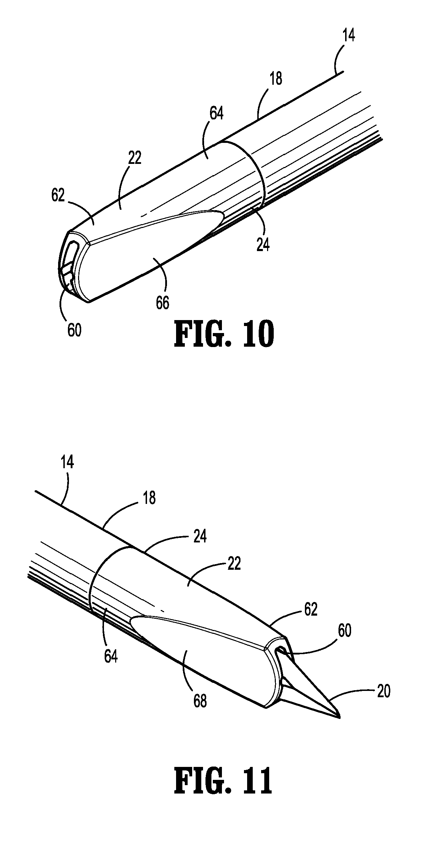

As shown in FIGS. 10 and 11, sheath 22 includes a slot 60 in a distal end 62 of sheath 22 and through which blade 20 is extended and retracted. As noted above, proximal end 24 is affixed to distal end 18 of elongate tubular member 14. Sheath 22 includes a cylindrical proximal portion 64 and tapered faces 66 (FIG. 10) and 68 (FIG. 11) extending distally from cylindrical proximal portion 64. Tapered faces 66 and 68 function as dissection surfaces to spread an incision in tissue created by blade 20.

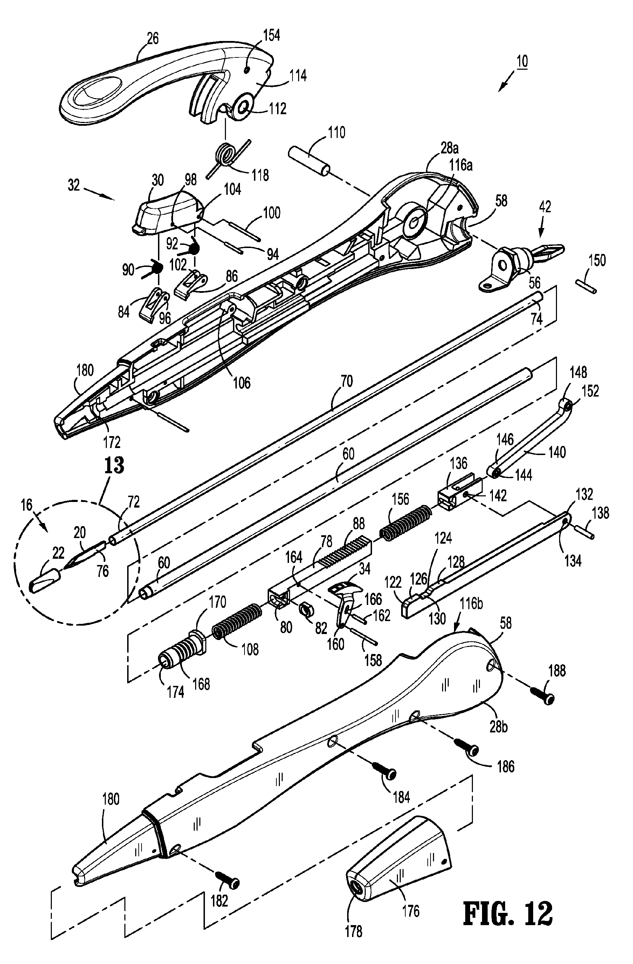

Turning now to FIG. 12, handle housing 28 of handle assembly 12 of surgical device 10 is formed as two complimentary halves 28a and 28b and are joined by welding, gluing, snap fit, etc. Connection assembly 42 extends through hole 58 formed in handle housing halves 28a and 28b and is secured therein by nut 56.

In order to move blade 20 between the extended and retracted positions in sheath 22, surgical device 10 includes an intermediate tube 70 having a distal end 72 and a proximal end 74. Distal end 72 of intermediate tube 70 is provided to receive a proximal end 76 of blade 20. Advancement mechanism 32 includes a hollow ratchet body 78 which is longitudinally mounted in handle housing 28 and includes a distal slot 80 for receipt of proximal end 74 of intermediate tube 70. A cam collar 82 secures proximal end 74 of intermediate tube 70 within distal slot 80 of hollow ratchet body 78.

Advancement mechanism 32 additionally includes an advance pawl 84 and a lock pawl 86 which are configured to engage ratchet teeth 88 provided on hollow ratchet body 78 and move hollow ratchet body 78 distally to extend blade 20 and lock it in position for use. First and second torsion springs 90 and 92 are provided between advance button 30 and advance pawl 84 and lock pawl 86 to bias the pawls into engagement with ratchet teeth 88 on hollow ratchet body 78. Advance pawl 84 is pivotally mounted to advance button 30 by a first pin 94 extending through holes 96 in advance pawl 84 and holes 98 in advance button 30. First pin 94 also supports first torsion spring 90. Likewise, a second pin 100 extends through holes 102 in lock pawl 86 and holes 104 in advance button 30. Second pin 100 supports second torsion spring 92. Additionally, second pin 100 serves to pivotally mount advance button 30 on handle housing 28 and is mounted within supports 106 in handle housing halves 28a and 28b. Thus, advance button 30 and lock pawl 86 have a common pivot point allowing lock pawl 86 to remain engaged with hollow ratchet body 78 until disengaged as described below. Hollow ratchet body 78 is biased proximally within handle housing 28 by a compression spring 108 to maintain blade 20 in a retracted position within sheath 22 (FIG. 10).

Lever 26 is operable to release advance pawl 84 and lock paw 86 from hollow ratchet body 78 and thus retract blade 20 within sheath 22. Lever 26 is pivotally connected to handle housing 28 by a pivot shaft 110 extending through a bore 112 formed in a base 114 of lever 26. Pivot shaft 110 extends through holes 116a and 116b formed in handle housing halves 28a and 28b, respectively. Lever 26 is biased to an open position by a torsion spring 118 supported by pivot shaft 110 and mounted in handle housing 28.

Surgical device 10 incorporates a cam release bar 120. Cam release bar 120 includes first and second cam surfaces or edges 122 and 124 for lifting advance pawl 84 and lock pawl 86, respectively, out of engagement with ratchet teeth 88 on hollow ratchet body 78. Relatively flat support surfaces 126 and 128 are located immediately proximal to cam edges 122 and 124 to maintain advance and lock pawls 84 and 86 out of engagement with hollow ratchet body 78 during operation of lever 26 to retract blade 20. A short depression 130 is located between support surfaces 126 and 128 to allow lock pawl 86 to engage hollow ratchet body 78 when cam release bar 120 is in a proximal position.

In order to drive cam release bar 120 against advance and lock pawls 84 and 86, a proximal end 132 of cam release bar 120 includes a hole 134 and is connected to a slider 136 by a pin 138. Slider 136 is connected through a link 140 to lever 26. Specifically, pin 138 extends through holes 142 in slider 136 and a bore 144 in a distal end 146 of link 140. A proximal end 148 of link 140 is connected to the lever 26 by a pin 150 which extends through a bore 152 in proximal end 148 and through holes 154 in lever 26. A compression spring 156 is provided within handle housing 28 and engages and biases slider 136 proximally within handle housing 28. Movement of lever 26 toward handle housing 28 drives link 140 and slider 136 distally within handle housing 28 to move cam bar 120 distally to retract blade 120 in a manner described in more detail hereinbelow.

Indicator 34 is provided to give a visual indication of the degree of extension of blade 20 out of sheath 22. Indicator 34 is pivotally connected to handle housing 28 by a pivot pin 158 which extends through a hole 160 in indicator 34. A drive pin 162 is affixed within a hole 164 in hollow ratchet body 78 and rides within a slot 166 formed in indicator 34. As hollow ratchet body 78 is advanced, indicator 34 is rotated about pivot pin 158 to display the position of blade 20 relative to sheath 22.

As shown, surgical device 10 additionally includes a guide sleeve 168 having a proximal flange170 which is supported in slots 172 formed in handle housing halves 28a and 28b. Guide sleeve 168 has a hollow bore 174 for support and passage of elongate tubular member 14. Guide sleeve is also connected to the tube 18 by, e.g., welding. Additionally, a conical end piece 176 having an opening 178 is affixed over an outer surface 180 of handle housing halves 28a and 28b to assist in holding them together. Elongate tubular member 14 passes through opening 178. In one embodiment, conical end piece 176 may be affixed to outer tubular member 14 and rotate relative to handle housing 28 to rotate and orient end effector assembly 16 relative to tissue.

Handle housing halves 28a and 28b are secured together by screws 182, 184, 186 and 188 allowing handle 28 to be dissembled for attachment and removal of connection assembly 42 and plug 50 (FIGS. 8 and 9).

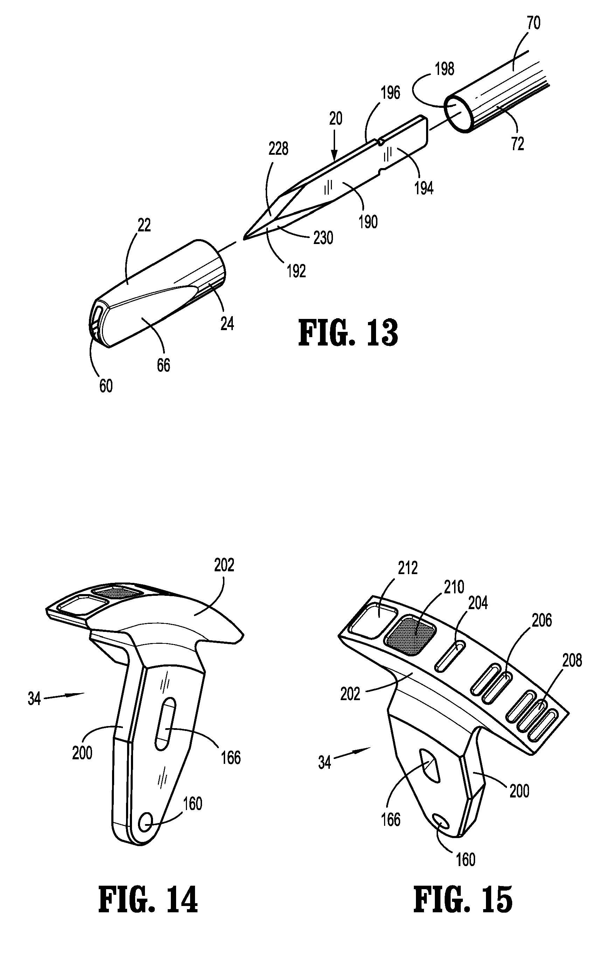

Referring now to FIG. 13, blade 20 is mounted for movement through slot 60 in sheath 22 and is driven by intermediate tube 70. Blade 20 generally includes a central body portion 190 having a sharp, pyramid shaped tissue penetrating distal tip 192. A reduced width proximal portion 194 extends proximally from a proximal end 196 of central body portion 190 and is rigidly mounted within a bore 198 in a distal 72 of intermediate tube 70 by known methods such as, for example, welding, gluing, etc. By mounting blade 20 rigidly to intermediate tube 70, advancement of blade 20 out of sheath 22 may be used to puncture tissue. Blade 20 may be formed from a variety of materials such as, for example, stainless steel, ceramics, polymers, etc. When connection assembly 42 is provided on surgical device 10 to provide cautery capabilities, blade 20 and intermediate tube 70 are formed from conductive, metallic materials. Sheath 22 is formed from an insulating material.

Turning now to FIGS. 14 and 15, indicator 34 includes a lever arm 200 and an arcuate head 202. Lever arm 200 includes pivot hole 160 for mounting indicator 34 on pivot pin 158 and is formed with slot 166 for receipt of drive pin 162 (FIG. 12). Arcuate head 202 includes blade position indicia such as, for example position indicia 204, 206 and 208. As hollow ratchet body 78 moves distally to extend blade 20, indicator 34 is rotated counter clockwise such that indicia 204, 206 and 208 become progressively visible through window 36 in handle housing 28. India 208 indicates full extension of blade 20 while indicia 206 and 204 indicate lesser degrees of partial extension of blade 20. In these three positions, blade 20 is extended. Arcuate head 202 is additionally provided with first and second safety indicia 210 and 212. First safety indicia 210 corresponds to a state where the tip of the blade tip 236 is flush with the distal tip of the sheath 22. Second safety indicia 212 corresponds to the condition wherein hollow ratchet body 78 is in a proximal most position securing blade 20 subflush within sheath 22 as described in more detail hereinbelow.

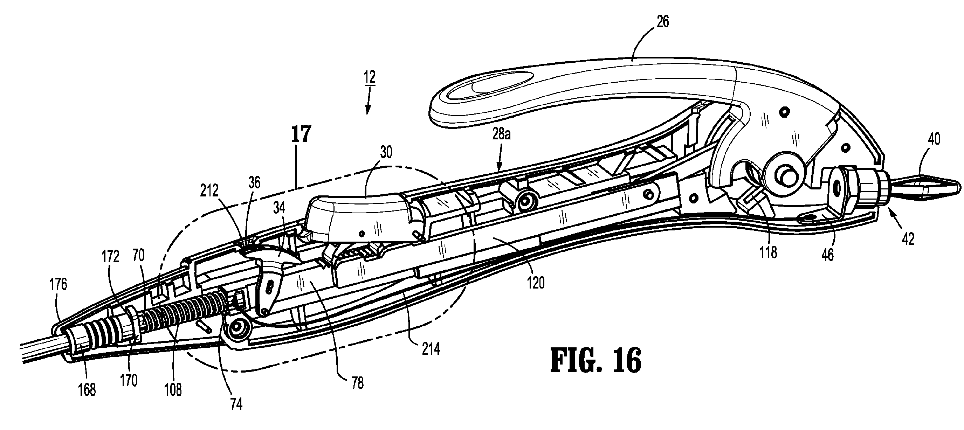

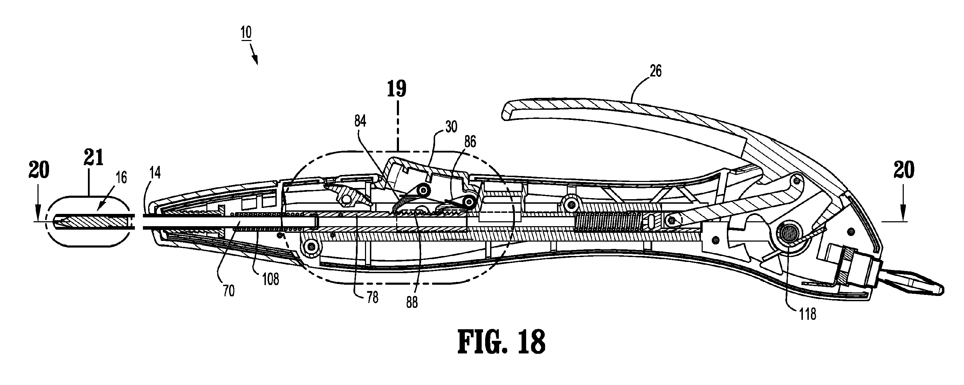

Turning now to FIGS. 16-31, and initially with regard to FIG. 16 the operation of surgical device 10 will now be described. Lever 26 is in the open position against the bias of compression spring 156 (FIG. 12) and torsion spring 118. Hollow ratchet body 78 is in a proximal most position retaining blade 20 within sheath 22 (FIG. 18). Advance button 30 is in the upper most position and available to advance blade 20. Indicator 34 is positioned such that second safety indicia 212 is visible through window 36 in handle housing 28.

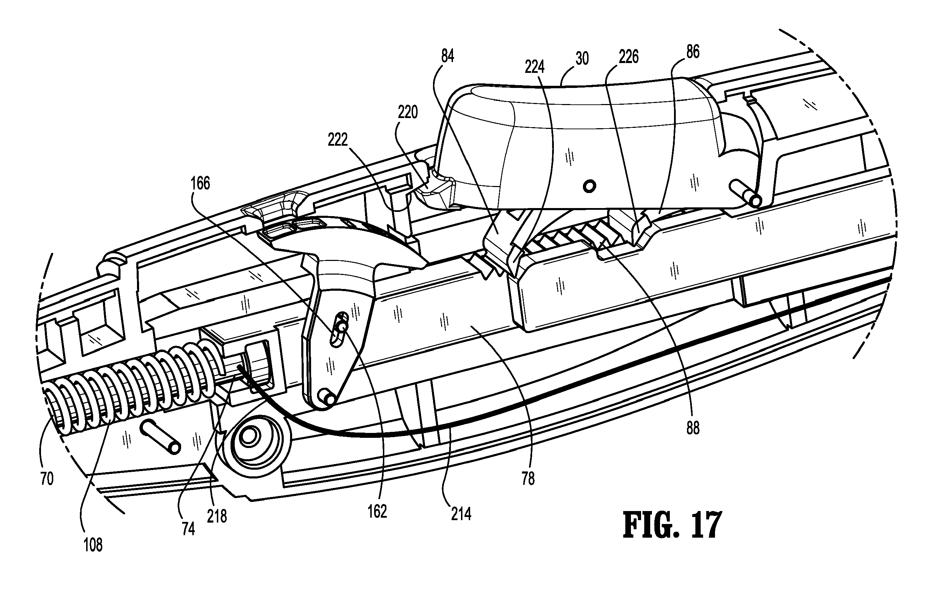

Referring for the moment to FIGS. 16 and 17, as noted above, surgical device 10 includes cautery capabilities to cauterize tissue cut by blade 20. A wire 214 extends from connection assembly 42 to intermediate tube 70. Specifically, a proximal end 216 of wire 214 is connected to connection plate 46 of connection assembly 42 and a distal end 218 of wire 214 is connected to proximal end 74 of intermediated tube 70. Blade 20 and intermediate tube 70 are electrically insulated from the remainder of surgical device 10. Thus, attachment of an electrical source (not shown) to cauterization connector 40 of connection assembly 42 transmits power through wire 214 and intermediate tube 70 to blade 20 for cauterizing of tissue.

Referring now to FIGS. 17 - 18, in the initial position, advance button 30 is in the upper position with advance pawl 84 and lock pawl 86 engaging ratchet teeth 88 of hollow ratchet body 78. Hollow ratchet body 78 and intermediate tube 70 (and thus blade 20) are maintained in the proximal position by compression spring 108. As best shown in FIG. 17, drive pin 162 in hollow ratchet body 78 lies within drive slot 166 of indicator 34 and maintains indicator 34 in the initial position indicating both advance button 30 is free to move and blade 20 is sheathed in sheath 22

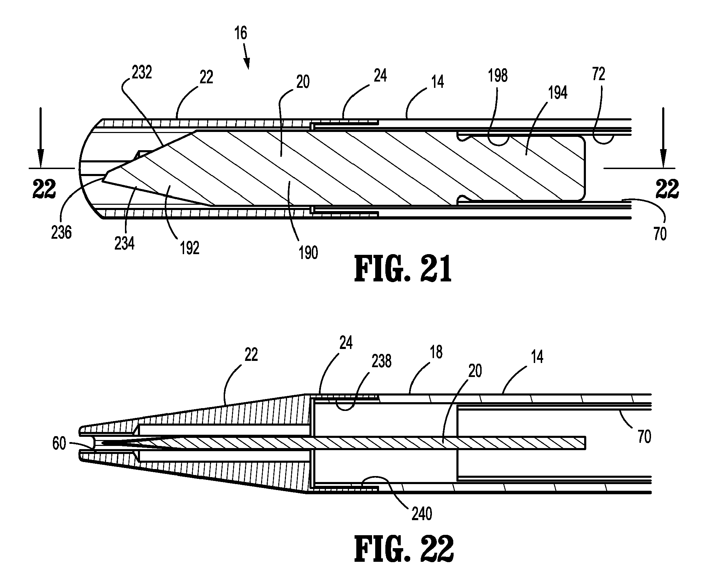

(FIGS. 18 and 21). Advance button 30 includes a distal lip 220 which engages a housing edge 222 to prevent advance button 30 from lifting out of handle housing 28.

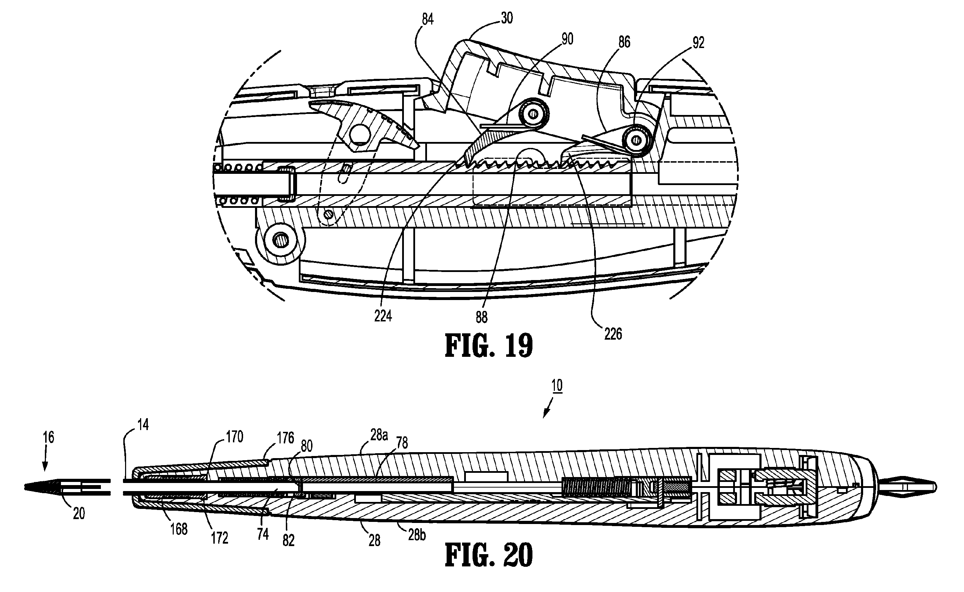

With specific reference to FIGS. 17 and 19, advance pawl 84 and lock pawl 86 include respective engagement teeth 224 and 226 which engage ratchet teeth 88 of hollow ratchet body 78. Engagement teeth 224 and 226 are biased into engagement with ratchet teeth 88 by respective first and second torsion springs 90 and 92 (FIG. 19). Additionally, first and second torsion springs 90 and 92 bias and maintain upward pressure on advance button 30.

Referring to FIG. 20, and as noted herein above, end effector assembly 16 is rotatable relative to handle housing 28 about the long axis of elongate tubular member 14 by manipulation of conical end piece 176. To accomplish this, guide sleeve 168 is affixed to elongate tubular member 14 and flange 170 of guide sleeve 168 is journaled or rotatably mounted within slots 172 in handle housing halves 28a and 28b. Conical end piece 176 is affixed to guide sleeve 168 (FIGS. 16 and 20). Proximal end 74 of intermediate tube 70 includes cam collar 82 which is rotatably mounted within distal slot 80 of hollow ratchet body 78. Thus, end effector assembly 16, and specifically blade 20, is free to rotate elongate tubular member 14.

Referring now to FIGS. 13, 21 and 22, blade 20 includes sharp, tissue cutting side edges 228 and 230 (FIG. 13). As best shown in FIG. 21, blade 20 additionally includes an upper surface 232, a lower surface 234 and an angled, tissue penetrating distal tip 236. Sheath 22 includes a recessed proximal bore 238 in proximal portion 24 which fits over and is affixed to a reduced diameter distal portion 240 in distal end 18 of elongate tubular member 14. While not specifically shown, blade 20 can include markings or indicia to provide a visual indication of the degree of extension of blade 20 out of sheath 22 or depth of penetration in tissue.

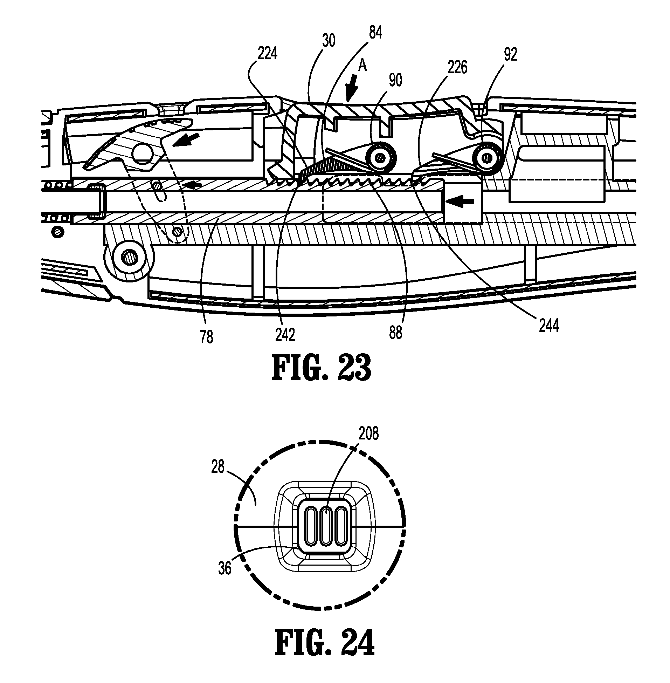

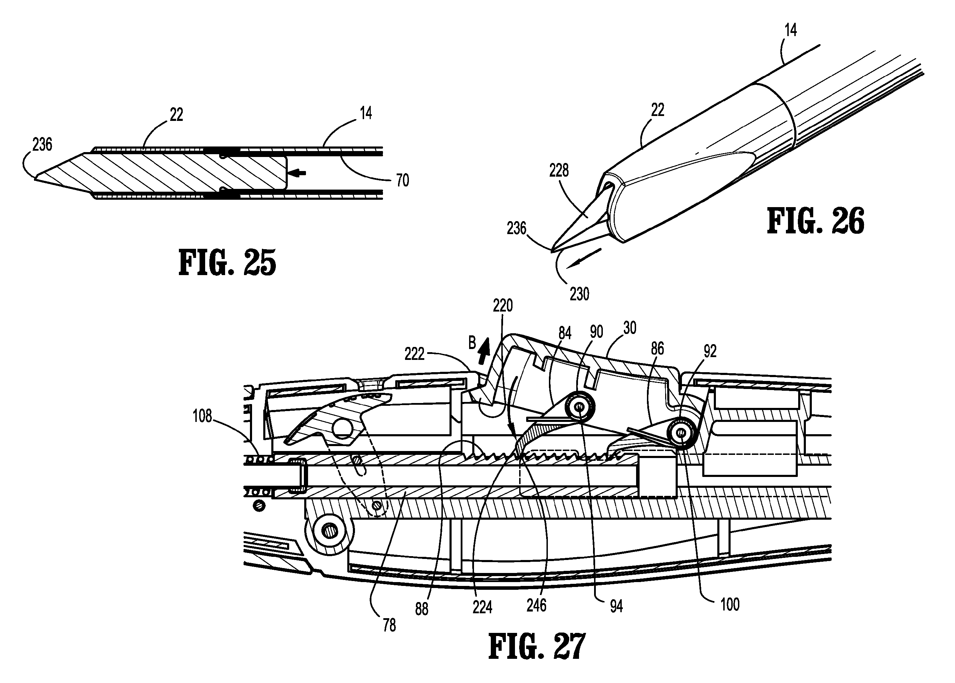

Turning now to FIGS. 23-31, and initially with regard to FIGS. 23-27, the movement of the various components of surgical device 10 in operation will now be described. Referring to FIG. 23 advance button 30 is depressed to extend blade 20. Specifically, advance button 30 is depressed in the direction of arrow "A" against the bias of torsion springs 90 and 92 causing advance pawl 84 to drive hollow ratchet body 78 distally within handle housing 28 (FIG. 23). More specifically, engagement tooth 224 of advance pawl 84 engages and drives a first or distal tooth 242 of ratchet teeth 88 on hollow ratchet body 78. Engagement tooth 226 of lock pawl 86 rides along ratchet teeth 88 and drops into engagement with a tooth 244 of hollow ratchet body 78 to block proximal movement of hollow ratchet body 78 and locking blade 20 in an extended position out of sheath 22 (FIGS. 25 and 26). Distal movement of hollow ratchet body 78 additionally rotates indicator 34 such that position indicia, such as, for example, position indicia 208 is visible though window 36 in handle housing 28 (FIG. 24).

Referring to FIGS. 23, 25 and 26 distal movement of hollow ratchet body 78 (FIG. 23) drives intermediate tube 70 distally within elongate tubular member 14 to advance blade 20 a first distance out of sheath 22 (FIGS. 25 and 26). This makes sharp, angled distal tip 236 and portions of cutting side edges 228 and 230 available for dissecting tissue.

Referring to FIG. 27, as pressure is released from advance button 30, advance button is biased upward in the direction of arrow "B" by first and second torsion springs 90 and 92. This arcuate movement of advance button 30 about first pin 94 draws engagement tooth 224 of advance pawl 84 proximally along ratchet teeth 88 of hollow ratchet body 78. Lock pawl 86 remains engaged with hollow ratchet body 78 due to the bias of torsion spring 92 and common pivot about pin 100. Lock pawl 86 maintains hollow ratchet body 78 in a distal position against the proximal bias of compression spring 108. As advance button returns to its full initial height, engagement tooth 224 of advance pawl 84 drops into engagement with a second more proximal tooth 246 and is again in position to advance hollow ratchet body 78 to extend blade 20 further out of sheath 22.

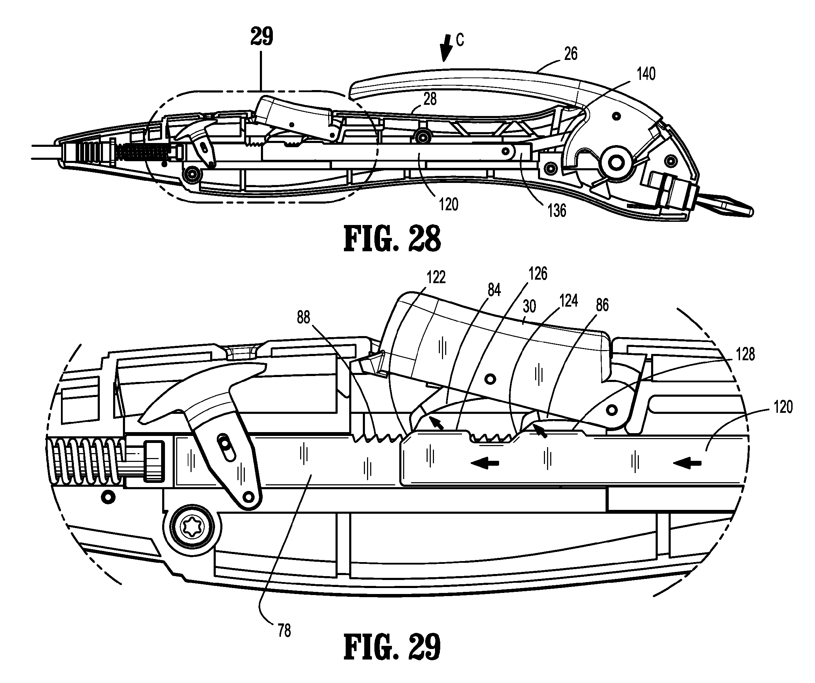

Referring now to FIG. 28, in order to release advance pawl 84 and, more particularly, lock pawl 86 from hollow ratchet body 78, lever 26 is depressed or compressed toward handle housing 28 in the direction of arrow "C". Compression of lever 26 against handle housing 28 causes drive link 140 to urge slider 136 distally within handle housing 28 against the bias of compression spring 156 (FIG. 12). As slider 136 moves distally, it advances cam release bar 120 distally. With reference to FIG. 29, as cam release bar 120 moves distally, first and second cam edges 122, 124 lift advance and lock pawls 84 and 86 out of engagement with ratchet teeth 88 on hollow ratchet body 78. Support surfaces 126 and 128 of cam release bar 120 maintain advance and lock pawls 84 and 86 out of engagement with hollow ratchet body 78.

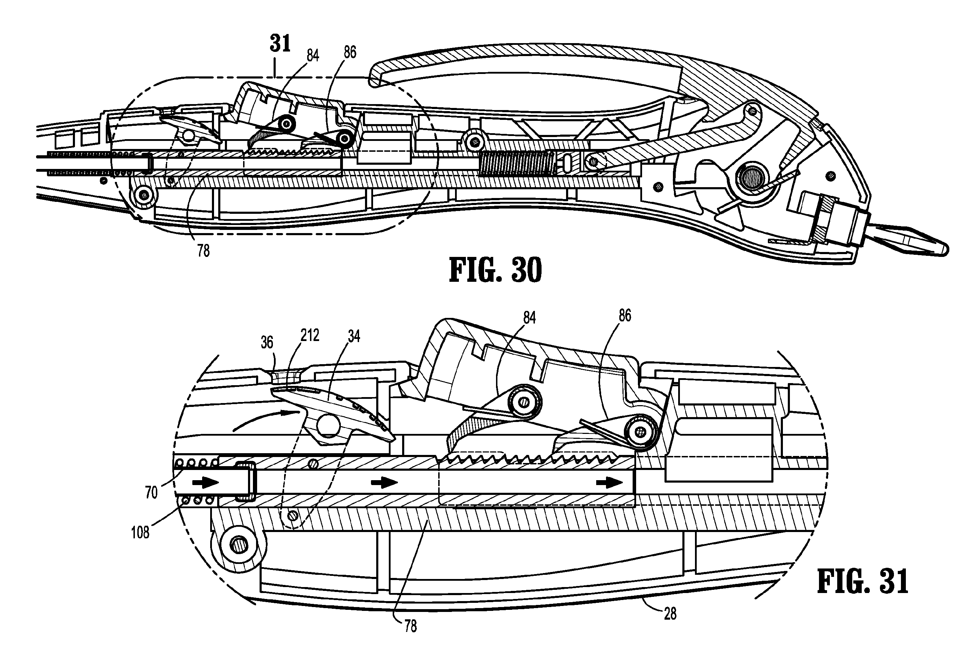

Referring to FIGS. 30 and 31, once advance and lock pawls 84 and 86 are out of engagement with hollow ratchet body 78, hollow ratchet body 78 is biased proximally by compression spring 108 to its proximal most position within handle housing 28. This draws intermediate tube 70 proximally thereby drawing blade 20 back into sheath 22 (FIG. 22). Additionally, proximal movement of hollow ratchet body 78 rotates indicator 34 back to the initial position to again display safety indicator 212 through window 36 in handle housing 28.

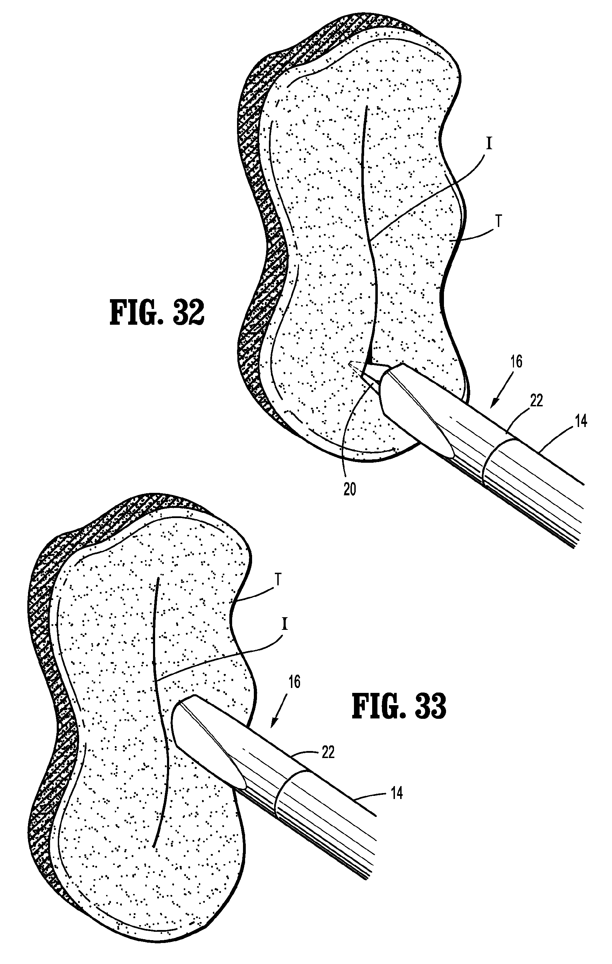

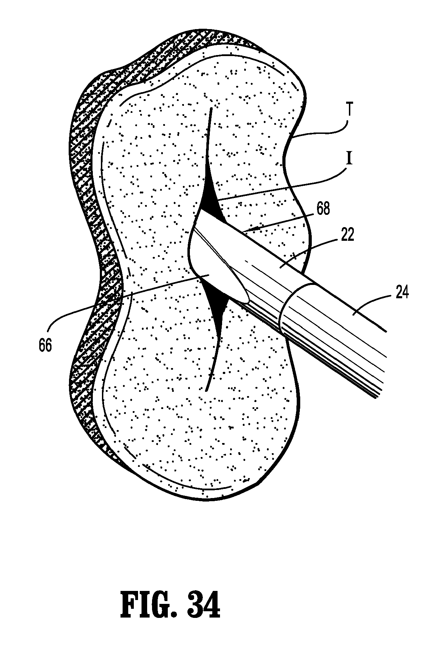

In this manner, surgical device 10 is able to dissect, cauterize and manipulate tissue. For example, with reference to FIGS. 1 and 32-34, surgical device 10 is manipulated to position end effector 16 adjacent a tissue T. Blade 20 is extended by actuation of advance button 30 and utilized to create an incision I in tissue T (FIG. 32). Blade 20 may be energized to cauterize incision I by supplying a suitable energy source to cauterization connector 40 (FIG. 1). Blade 20 is then retracted by depressing lever 26 (FIGS. 1 and 33). End effector 16 may then be reinserted into incision I created in tissue T. Tapered faces 66 and 68 of sheath 22 are used to force incision I apart to create an opening for further insertion of surgical instruments.

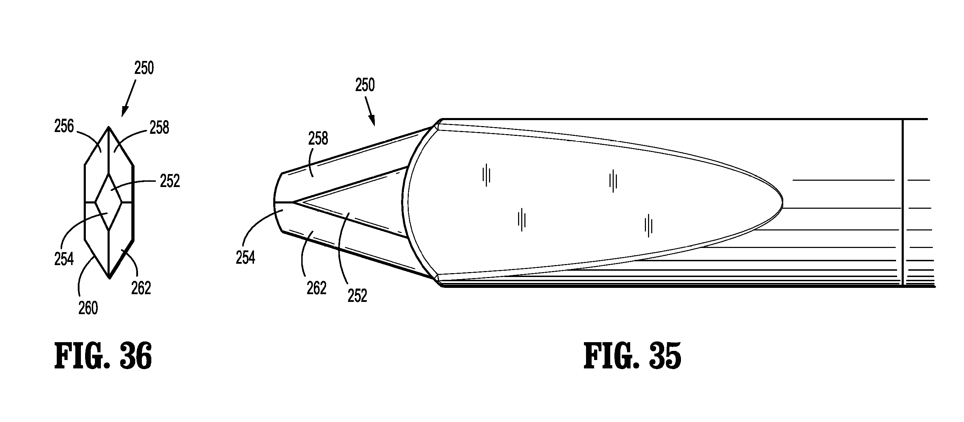

Referring to FIGS. 35 and 36, an alternate blade 250 may be provided for use with surgical device 10. Blade 250 has a symmetrical tissue dissecting tip 252. Body portion 252 additionally includes distal tip 254 and tissue cutting side edges 256 and 258 and 260 and 262. Here the distal tip is blunt to prevent perforation of the pyloric canal mucosa.

It will be understood that various modifications may be made to the embodiments disclosed herein. For example, other blade geometries may be provided including differing cutting edges, blunt dissection surfaces, etc. Further, as noted hereinabove, indicia may be provided on blade surfaces to indicate degree of extension out of the sheath and may correspond to indicia on the indicator. Additionally, other rigid connections may be provided between the blade and the intermediate tube or driving member. Therefore, the above description should not be construed as limiting, but merely as exemplifications of particular embodiments. Those skilled in the art will envision other modifications within the scope and spirit of the claims appended hereto.

The invention may be described by reference to the following numbered paragarphs:-
1. A surgical device for use in pediatric surgery comprising:
   a handle assembly having a handle housing,
   an elongate tubular member extending distally from the handle assembly and having a distal end, and
   an end effector assembly mounted on the distal end of the elongate tubular member, the end effector assembly including a tissue dissecting sheath and a blade movably mounted within the sheath.
2. The surgical device as recited in paragraph 1, wherein the handle assembly includes an advancement mechanism for extending the blade out of the sheath, the advancement mechanism being connected to the blade by an intermediate tube rigidly affixed to the blade and movably mounted within the elongate tubular member for incremental longitudinal movement of the blade.
3. The surgical device as recited in paragraph 2, wherein the advancement mechanism includes a body connected to a proximal end of the intermediate tube and an advance button movably mounted in the handle housing and engageable with the body to drive the hollow body distally within the handle assembly.
4. The surgical device as recited in paragraph 3, wherein the advancement mechanism includes an advance pawl rotatably mounted to the advance button and the body includes ratchet teeth engageable by the advance pawl to incrementally advance the body distally within the handle housing in response to depression of the advance button against the handle housing.
5. The surgical device as recited in paragraph 4, wherein the advance button is pivotally mounted on the handle housing at a pivot point and the advancement mechanism further includes a lock pawl engageable with the ratchet teeth and sharing the pivot point with the advance button such that the lock pawl remains engaged with the ratchet teeth upon release of the advance button.
6. The surgical device as recited in paragraph 5, further comprising a cam release bar movably mounted within the handle housing and connected to a lever, the cam release bar having a first cam edge engageable with the advance pawl and a second cam edge engageable with the lock pawl such that actuation of the lever drives the cam release bar distally within the housing to lift the advance pawl and lock pawl out of engagement with the ratchet teeth.
7. The surgical device as recited in paragraph 1, further comprising a cauterization assembly including an electrical connection assembly mounted on a proximal end of the handle housing, the electrical connection assembly providing cauterizing current to the blade.
8. The surgical device as recited in paragraph 7, wherein the cauterization assembly further includes a wire extending from the electrical connection assembly through the housing to the blade.
9. A surgical device for use in pediatric surgery comprising:
   a handle assembly having a handle housing,
   an elongate tubular member extending distally from the handle assembly and having a distal end,
   an end effector assembly mounted on the distal end of the elongate tubular member, the end effector assembly including a sheath and a blade movably mounted within the sheath,
   an advancement mechanism positioned within the handle housing and operable to extend the blade out of the sheath; and
   a lever movably mounted on the handle housing, the lever being operable to release the blade from an extended position relative to the sheath to a retracted position within the sheath.
10. The surgical device as recited in paragraph 9, wherein the blade is biased proximally within the sheath by a compression spring.
11. The surgical device as recited in paragraph 10, wherein the blade is rigidly connected to an intermediate tube.
12. The surgical device as recited in paragraph 11, wherein a proximal end of the intermediate tube is connected to a ratchet body movably mounted in the handle housing.
13. The surgical device as recited in paragraph 12, wherein the compression spring biases the ratchet body proximally within the handle housing.
14. The surgical device as recited in paragraph 13, wherein the advancement mechanism includes an advance pawl engageable with the ratchet body.
15. The surgical device as recited in paragraph 14, wherein the advancement mechanism includes a lock pawl engageable with the ratchet body.
16. The surgical device as recited in paragraph 15, further comprising a cam release bar movably mounted in the handle housing and engageable with the advance and lock pawls to disengage the advance and lock pawls from the ratchet body.
17. The surgical device as recited in paragraph 16, wherein the cam release bar is connected to the lever by a linkage.
18. A surgical device for use in pediatric surgery comprising:
   a handle assembly having a handle housing,
   an elongate tubular member extending distally from the handle assembly and having a distal end,
   an end effector assembly mounted on the distal end of the elongate tubular member, the end effector assembly including a sheath and a blade movably mounted within the sheath,
   an advancement mechanism positioned within the handle housing and operable to extend the blade out of the sheath the advancement mechanism including a ratchet body rigidly connected to the blade, an advance button mounted to the handle housing and an advance pawl connected to the advance button and engageable with teeth on the ratchet body;
   a lock pawl connected to the advance button and engageable with the ratchet body to maintain the ratchet body and blade in a distal position;
   a lever movably mounted on the handle housing and a cam release bar connected to the lever, the lever operable to drive the cam release bar against the lock pawl to release the blade for proximal movement within the sheath; and
   an electrical connector on the handle housing for supplying cauterizing current to the blade.
19. The surgical device as recited in paragraph 18, wherein the ratchet body is rigidly connected to the blade by an intermediate rod.
20. The surgical device as recited in paragraph 19, further comprising a wire extending from the electrical connector to a proximal end of the intermediate rod.

## Claims

1. A surgical device for use in pediatric surgery comprising:
a handle assembly having a handle housing,
an elongate tubular member extending distally from the handle assembly and having a distal end, and
an end effector assembly mounted on the distal end of the elongate tubular member, the end effector assembly including a tissue dissecting sheath and a blade movably mounted within the sheath.

2. The surgical device as recited in claim 1, wherein the handle assembly includes an advancement mechanism for extending the blade out of the sheath, the advancement mechanism being connected to the blade by an intermediate tube rigidly affixed to the blade and movably mounted within the elongate tubular member for incremental longitudinal movement of the blade, preferably wherein the advancement mechanism includes a body connected to a proximal end of the intermediate tube and an advance button movably mounted in the handle housing and engageable with the body to drive the hollow body distally within the handle assembly.

3. The surgical device as recited in claim 2, wherein the advancement mechanism includes an advance pawl rotatably mounted to the advance button and the body includes ratchet teeth engageable by the advance pawl to incrementally advance the body distally within the handle housing in response to depression of the advance button against the handle housing, preferably wherein the advance button is pivotally mounted on the handle housing at a pivot point and the advancement mechanism further includes a lock pawl engageable with the ratchet teeth and sharing the pivot point with the advance button such that the lock pawl remains engaged with the ratchet teeth upon release of the advance button.

4. The surgical device as recited in claim 3, further comprising a cam release bar movably mounted within the handle housing and connected to a lever, the cam release bar having a first cam edge engageable with the advance pawl and a second cam edge engageable with the lock pawl such that actuation of the lever drives the cam release bar distally within the housing to lift the advance pawl and lock pawl out of engagement with the ratchet teeth.

5. The surgical device as recited in any preceding claim, further comprising a cauterization assembly including an electrical connection assembly mounted on a proximal end of the handle housing, the electrical connection assembly providing cauterizing current to the blade, preferably wherein the cauterization assembly further includes a wire extending from the electrical connection assembly through the housing to the blade.

6. A surgical device for use in pediatric surgery comprising:
a handle assembly having a handle housing,
an elongate tubular member extending distally from the handle assembly and having a distal end,
an end effector assembly mounted on the distal end of the elongate tubular member, the end effector assembly including a sheath and a blade movably mounted within the sheath,
an advancement mechanism positioned within the handle housing and operable to extend the blade out of the sheath; and
a lever movably mounted on the handle housing, the lever being operable to release the blade from an extended position relative to the sheath to a retracted position within the sheath.

7. The surgical device as recited in claim 6, wherein the blade is biased proximally within the sheath by a compression spring, preferably wherein the blade is rigidly connected to an intermediate tube.

8. The surgical device as recited in claim 7, wherein a proximal end of the intermediate tube is connected to a ratchet body movably mounted in the handle housing.

9. The surgical device as recited in claim 7 or claim 8, wherein the compression spring biases the ratchet body proximally within the handle housing.

10. The surgical device as recited in any of claims 6 to 9 wherein the advancement mechanism includes an advance pawl engageable with the ratchet body, preferably wherein the advancement mechanism includes a lock pawl engageable with the ratchet body.

11. The surgical device as recited in claim 10, further comprising a cam release bar movably mounted in the handle housing and engageable with the advance and lock pawls to disengage the advance and lock pawls from the ratchet body.

12. The surgical device as recited in claim 11, wherein the cam release bar is connected to the lever by a linkage.

13. A surgical device for use in pediatric surgery comprising:
a handle assembly having a handle housing,
an elongate tubular member extending distally from the handle assembly and having a distal end,
an end effector assembly mounted on the distal end of the elongate tubular member, the end effector assembly including a sheath and a blade movably mounted within the sheath,
an advancement mechanism positioned within the handle housing and operable to extend the blade out of the sheath the advancement mechanism including a ratchet body rigidly connected to the blade, an advance button mounted to the handle housing and an advance pawl connected to the advance button and engageable with teeth on the ratchet body;
a lock pawl connected to the advance button and engageable with the ratchet body to maintain the ratchet body and blade in a distal position;
a lever movably mounted on the handle housing and a cam release bar connected to the lever, the lever operable to drive the cam release bar against the lock pawl to release the blade for proximal movement within the sheath; and
an electrical connector on the handle housing for supplying cauterizing current to the blade.

14. The surgical device as recited in claim 13, wherein the ratchet body is rigidly connected to the blade by an intermediate rod.

15. The surgical device as recited in claim 14, further comprising a wire extending from the electrical connector to a proximal end of the intermediate rod.
